# EUROPEAN PATENT SPECIFICATION

(11) **EP 3 854 423 B1**
(45) Date of publication and mention of the grant of the patent: **09.08.2023**
(21) Application number: 18934139.9
(22) Date of filing: 12.10.2018
(51) Int. Cl.: A61L 27/20

(54) **ARTIFICIAL DERMIS REPAIR MATERIAL AND PREPARATION METHOD THEREFOR**
REPARATURMATERIAL FÜR KÜNSTLICHE DERMIS UND HERSTELLUNGSVERFAHREN DAFÜR
MATÉRIAU DE RÉPARATION DE DERME ARTIFICIEL ET MÉTHODE DE PRÉPARATION DE CELUI-CI

(30) Priority: 19.09.2018 CN 201811096057
(43) Date of publication of application: 28.07.2021
(73) Proprietor: Shenzhen Tsingcare Medical Instruments Co., Ltd., Shenzhen, Guangdong 518107 (CN)
(72) Inventor: WANG, Danyan, Shenzhen, Guangdong 518107 (CN); TAN, Rongwei, Shenzhen, Guangdong 518107 (CN); SHE, Zhending, Shenzhen, Guangdong 518107 (CN); GUO, Yuanjun, Shenzhen, Guangdong 518107 (CN); LIU, Xi, Shenzhen, Guangdong 518107 (CN); XU, Mengqiang, Shenzhen, Guangdong 518107 (CN); CHEN, Yingying, Shenzhen, Guangdong 518107 (CN)
(74) Representative: J A Kemp LLP
(86) International application number: PCT/CN2018/110016
(87) International publication number: WO 2020/056815

(56) References cited:
- EP-A1- 3 015 091
- CN-A- 1 785 444
- CN-A- 106 693 075
- CN-A- 106 853 263
- CN-A- 107 899 080
- JP-A- H04 266 764
- US-A- 4 060 081
- US-A1- 2006 008 905
- US-A1- 2016 143 726
- US-A1- 2016 317 625

## Description

### TECHNICAL FIELD

The present disclosure relates to the field of medical instruments and the field of tissue engineering regeneration, and in particular, to an artificial dermal repair material and a preparation method thereof.

### BACKGROUND

Skin is the largest organ of a human body, and can protect the body from harmful substances from outside, prevent the loss of water, electrolytes, and the like in the body, maintain the stability of an internal environment of the human body, and participate in immune and metabolic processes of the human body. Skin tissue defects caused by burns, diseases, physical trauma, wars, and the like affect the quality of life of people and even threaten lives. According to a report by the World Health Organization (WHO) (January 2018), 180,000 people die of burns worldwide each year. In 2004, nearly 11 million people worldwide were severely burned and required medical attention. In 2000, direct medical expenditure on burns for children in America exceeded $211 million. In 2007, hospital burn management expenditure in Norway exceeded €10.5 million. Indirect losses such as lost wages, long-term care for disabilities and psychological trauma, and invested household resources have also exacerbated the impact on society and economy. When the skin is damaged in a large area or severely, the skin cannot repair itself and needs to be repaired by skin grafts/skin flaps or dermal substitute transplantation. At present, dermal substitutes for treating a wound surface mainly include artificial dermis (made of biological and synthetic materials), allogeneic or xenogeneic natural dermis (treated by decellularization), tissue engineering active dermis (such as Apligraf^{®}, IERFU^{®}), etc. Artificial dermal substitute products have no limitation on material sources and ethics, have low virus infection risk, are easy to store and transport, can be produced in batches, have increasingly mature production, safety, and quality control, and have low cost, thereby gradually occupying the mainstream of market development.

Collagen, as the most commonly used biological material, is widely used to prepare artificial skin. For example, both Integra^{®} and Lando^{®} artificial dermis that have been approved for marketing use collagen-glycosaminoglycan (GAG) materials and are clinically used for deep burns and full-thickness skin defects. US Patent US 20160143726 A1 discloses a method of preparing a collagen-GAG-elastin scaffold, and the preparation process thereof includes preparing a collagen-GAG-elastin complex solution, freeze-drying the complex solution into a porous scaffold, and then performing cross-linking. These scaffolds can be used in the medical and surgical fields, such as the reconstruction of dermal tissue. However, existing artificial dermal substitutes still cannot meet the requirements of ideal dermal substitutes, and have problems such as vascularization, wound infection, and the like in clinical applications. Therefore, it is necessary to invent materials with better physical properties and antibacterial properties for use in the field of skin tissue engineering.

### SUMMARY

A technical problem to be solved by the present disclosure is to provide an artificial dermal repair material and a preparation method thereof. The prepared artificial dermal repair material has good physical properties and antibacterial properties, and is applicable in the field of skin tissue engineering.

The present disclosure adopts technical solutions as follows.

The present disclosure provides an artificial dermal repair material, which includes a silicone rubber layer and a collagen complex layer bonded to each other. The collagen complex layer is prepared by raw materials including collagen, glycosaminoglycan, and an antibacterial agent by a cross-linking reaction.

The silicone rubber layer includes a first silicone rubber layer, a second mesh-shaped silicone rubber intermediate layer, and a third silicone rubber layer which are provided in sequence. A tensile strength of the second mesh-shaped silicone rubber intermediate layer is greater than a tensile strength of the first silicone rubber layer and a tensile strength of the third silicone rubber layer.

In some more preferred embodiments, the tensile strength of the first silicone rubber layer and/or the third silicone rubber layer is less than 2 MPa. The tensile strength of the second mesh-shaped silicone rubber intermediate layer is greater than 10 MPa.

In some preferred embodiments, the collagen complex layer is of a gradient porous structure. Further, sizes of holes in the gradient porous structure are distributed in a gradient manner, and a hole size increases from 50 µm to 250 µm.

In some preferred embodiments, the raw materials include 73% to 97% of the collagen, 2% to 15% of the glycosaminoglycan, and 1% to 12% of the antibacterial agent.

In some preferred embodiments, the raw materials include 78% to 93% of the collagen, 3% to 10% of the glycosaminoglycan, and 4% to 12% of the antibacterial agent.

In some preferred embodiments, the glycosaminoglycan is chondroitin sulfate or hyaluronic acid.

In some preferred embodiments, the antibacterial agent is at least one selected from the group consisting of polyhexamethylene biguanide (PHMB), lysozyme, and lysostaphin.

In some preferred embodiments, at least one of the silicone rubber layer and the collagen complex layer is provided with a plurality of holes.

In some more preferred embodiments, diameters of the holes on the silicone rubber layer are in a range of 50 µm to 1000 µm. Spacing of the holes are in a range of 0.5 mm to 10 mm.

In some more preferred embodiments, diameters of the holes on the collagen complex layer are in a range of 50 µm to 500 µm. Spacing of the holes are in a range of 0.5 mm to 5 mm.

In some preferred embodiments, the collagen complex layer has a thickness of 1 mm to 3 mm.

In some preferred embodiments, the silicone rubber layer has a thickness of 0.2 mm to 0.3 mm.

The present disclosure further provides a method of preparing the artificial dermal repair material as described above, which includes:
preparing a collagen complex layer, including:
dissolving glycosaminoglycan in a solvent to obtain a glycosaminoglycan solution, and dissolving collagen in a solvent to obtain a collagen solution;
mixing the glycosaminoglycan solution and the collagen solution, performing centrifugation and collecting a precipitate, and redissolving the precipitate by adding acetic acid to obtain a collagen-glycosaminoglycan complex solution; and
adding a cross-linking agent and an antibacterial agent into the collagen-glycosaminoglycan complex solution, performing freeze-drying, and then further performing a cross-linking reaction under a vacuum condition, to obtain the collagen complex layer;
preparing a silicone rubber layer,
bonding the collagen complex layer and the silicone rubber layer, and
optionally preparing a hole on at least one of the collagen complex layer and the silicone rubber layer.

In some preferred embodiments, the cross-linking reaction is further performed under a high-temperature vacuum condition of 100°C to 120°C.

The preparing the silicone rubber layer includes: mixing and curing materials of a first silicone rubber to form the first silicone rubber layer; mixing materials of a second silicone rubber to form a mixed solution, preparing a mesh-shaped layer on the first silicone rubber layer with the mixed solution by using a dispenser, and performing curing to form a second mesh-shaped silicone rubber intermediate layer; and leveling materials of a third silicone rubber on a surface of the second mesh-shaped silicone rubber intermediate layer, and performing curing to form a third silicone rubber layer. A tensile strength of the materials of the second silicone rubber after film formation is greater than a tensile strength of the materials of the first silicone rubber and a tensile strength of the materials of the third silicone rubber after film formation.

In some preferred implementations, the acetic acid has a concentration of 0.03M to 0.1M.

In some preferred embodiments, the performing freeze-drying includes: performing freezing under a low temperature environment, annealing for 5min to 10min, and then freezing under a low temperature environment, and then freeze-drying in a freeze dryer.

In further preferred embodiments, the low temperature environment has a temperature in a range of -50°C to 70°C.

In some preferred embodiments, the cross-linking agent is one selected from the group consisting of glutaraldehyde, genipin, and carbodiimide.

In some preferred embodiments, the cross-linking agent accounts for the collagen-glycosaminoglycan complex solution in a mass range of 0.005% to 0.015%.

In some preferred embodiments, the glycosaminoglycan solution is added to the collagen solution for mixing by high-speed airflow atomization.

In some preferred embodiments, the holes may be prepared by any of laser drilling, physical puncturing, and freeze-drying into holes in a special mold with regularly arranged protrusions at a bottom thereof.

In some preferred embodiments, the collagen complex layer and the silicone rubber layer are bonded together by silicone gel.

The beneficial effects achieved by the present disclosure are:
In the artificial dermal repair material provided by the present disclosure, the silicone rubber layer has high strength, high elasticity, and softness, is easy stitch, and has good fitting performance. The collagen complex layer is beneficial to the growth of fibroblasts, endothelial cells, and capillaries, and has good biocompatibility, degradability, and antibacterial property, which is applicable for the repair and reconstruction of dermal tissue.

### BRIEF DESCRIPTION OF THE DRAWINGS

FIG. 1 is an atomic force microscope (AFM) image of a collagen-chondroitin sulfate complex in Example 1.
FIG. 2 is a scanning electron microscope (SEM) image of the collagen-chondroitin sulfate complex in Example 1.
FIG. 3 is a SEM image showing a cross section of a collagen complex layer in Example 1.
FIG. 4 is a SEM image of a drilled collagen complex layer in Example 2.
FIG. 5 is photos showing results of bacteriostatic experiments in Example 3.
FIG. 6 is a photo showing a drilled silicone rubber layer in Example 4.

### DETAILED DESCRIPTION OF THE EMBODIMENTS

The invention is defined by the claims. Any subject-matter falling outside the scope of the claims is provided for information purposes only. In the following, the concept and the produced technical effects of the present disclosure will be clearly and completely described in conjunction with the embodiments to fully understand the purpose, features and effects of the present disclosure. Apparently, the described embodiments are only a part of the embodiments of the present disclosure, rather than all of them.

### Example 1

This example provides an artificial dermal repair material, and a preparation process thereof is as follows.

At S1, a collagen complex layer was prepared.

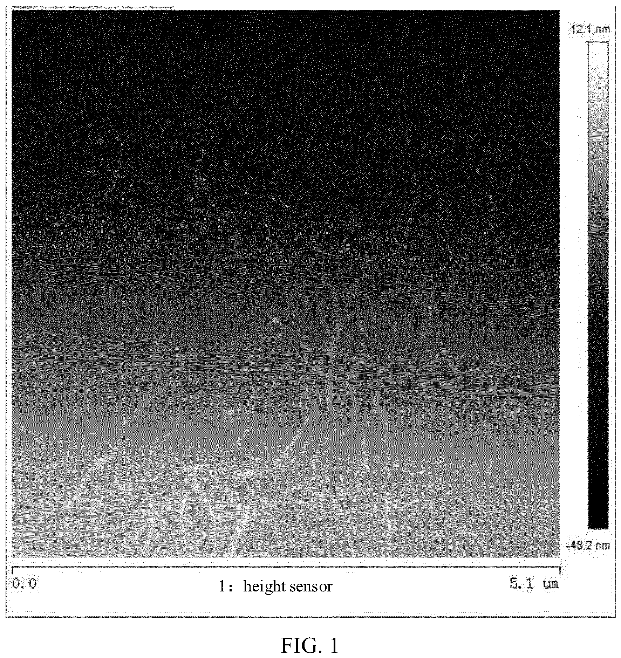

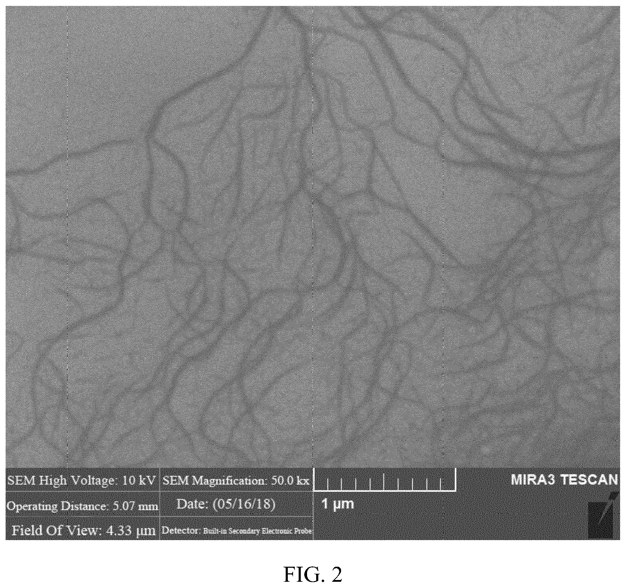

Collagen was weighed and dissolved in water to prepare 200 g of a collagen solution with a mass concentration of 0.3%. Then, 0.056 g of chondroitin sulfate was weighed and dissolved in water to prepare a chondroitin sulfate solution with a mass concentration of 1.7 mg/mL. The chondroitin sulfate solution was atomized by high-speed airflow and then added to the collagen solution, and continuously stirred for 2 hours to obtain a collagen-chondroitin sulfate complex. The collagen-chondroitin sulfate complex was centrifuged at 8000 rpm for 15 minutes. The precipitate was collected, and 0.05M acetic acid was added to re-dissolve the precipitate to make the concentration of collagen 0.9% (w/w), and then stirred for 2.5h to be dispersed uniformly, thereby forming a dispersion of the collagen-chondroitin sulfate complex. The dispersion of the collagen-chondroitin sulfate complex was characterized. An AFM image was as shown in FIG. 1 and a SEM image was as shown in FIG. 2. It can be seen from the figures that the obtained complex can be co-precipitated to form nano-scale collagen fibers, and the nano-scale collagen fibers can be self-assembled to form collagen fiber bundles, forming a uniformly dispersed collagen-chondroitin sulfate complex. A glutaraldehyde crosslinking agent solution (acetic acid as solvent) was added to make the concentration of the collagen 0.7% (w/w), where a mass fraction of the added glutaraldehyde was 0.01% (that is, a mass fraction of the glutaraldehyde accounting for the collagen complex solution), and then stirred for 5h to be uniformly distributed. Then, a polyhexamethylene biguanide (PHMB) antibacterial agent solution was added to make the concentration of the collagen 0.65% (w/w), where the amount of the added PHMB was 0.028g, and then was stirred for 2h to be uniformly distributed, and then was poured into an aluminum alloy mold, leveled, pre-frozen at -60°C for 2h, and then annealed for 5min to 10min, and then frozen at -60°C for half an hour, and then transferred to a freeze dryer for freeze-drying for 24h, and then placed under a high-temperature vacuum condition of 105°C for high-temperature cross-linking for 24 hours, to obtain the collagen complex layer.

At S2, a silicone rubber layer was prepared.

Components A and B of liquid silicone rubber with normal strength (with tensile strength <2Mpa after film formation) were mixed together in a ratio of 1:1, stirred at 300 rpm for 10 minutes, and then defoamed, and then poured into a polytetrafluoroethylene mold, leveled, and placed in a drying oven and cured at 150°C for 2 hours to obtain a first silicone rubber layer with a thickness of 0.12 mm.

Components A and B of liquid silicone rubber with high strength (with tensile strength >10Mpa after film formation) were mixed together in a ratio of 1:1, stirred at 300 rpm for 10 minutes to form a mixed solution, and then the mixed solution was defoamed. A layer of silicone rubber layer in a parallelogram mesh shape was uniformly prepared on a surface of the first silicone rubber layer with the mixed solution by using a dispenser, in which a spacing between parallel lines was 1mm to 10mm, and then was placed in a drying oven for curing, and cured at 150°C for 2 hours, to obtain a second mesh-shaped silicone rubber layer with a thickness of 0.02 mm.

Then, a liquid silicone rubber with normal strength (with tensile strength <2Mpa after film formation) was leveled on a surface of the second mesh-shaped silicone rubber layer, and then placed in a drying oven, and cured at 150°C for 2 hours, to obtain a third silicone rubber layer with a thickness of 0.12 mm. Finally, a silicone rubber layer with a thickness of 0.26 mm and having a mesh-shaped intermediate layer was formed.

At S3, a layer of silicone gel was coated on a surface of the silicone rubber layer, and a drilled collagen complex layer was bonded to a surface of the silicone rubber film to obtain the artificial dermal repair material.

At S4, packaging and sterilizing were performed. The prepared artificial dermal repair material was packaged, and sterilized by high-energy electron beam with a sterilization dose of 15KGy to 20KGy.

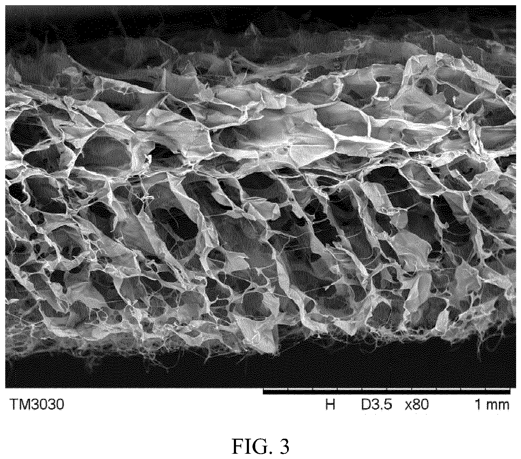

The collagen complex layer prepared in this example is characterized, and a SEM image showing a cross section of the collagen complex layer is as shown in FIG. 3. It can be seen from the figure that the prepared collagen complex layer has a three-dimensional gradient porous structure, and a pore size increases from 50 µm to 250 µm from a position in contact with the silicone rubber layer to a position away from the silicone rubber layer. In the prior art, the gradient porous structure is prepared generally by spraying and freeze-drying multiple layers of mixed solutions with different concentrations to achieve the effect of gradient pores, which has a more complicated preparation process, and the pore size increases suddenly. In the present disclosure, the gradient pores are prepared by pre-freezing, annealing, and freeze-drying, the operation is simple, the pore size increases transitionally and naturally, which is more beneficial to the growth of fibroblasts, endothelial cells, and capillaries.

The silicone rubber layer prepared in this implementation is measured for tensile strength. The specific steps of the measurement are as follows. Referring to the standard of GB/T 1040.3-2006 "Measurement of Tensile Properties of Plastics Part 3: Test Conditions for Films and Sheets", type 5 dumbbell-shaped test pieces are prepared by a cutter, respectively, and 3 parallel test pieces are prepared for each group of samples. A specified marking is marked on the test piece. A thickness *h* and a width *b* of each test piece are measured within 5 mm from each end of the gauge length in the middle of each test piece. 3 points are measured to calculate the average value. The electronic universal testing machine is turned on, test parameters are set, and related test parameters such as thickness and gauge length are input. The test speed was set to 500 mm/min. The sensor is 1000 N. The test pieces are mounted on the fixture so that long axes of the test pieces are in line with an axis of the testing machine. The run button is clicked to test. The tensile strength is calculated according to the following formula.

Tensile strength calculation formula: aM=F/S. In the formula: σM: tensile strength in MPa; F: force (maximum value) required for tearing the test piece, in N; S: original cross-sectional area of the test piece, mm². The test results are shown in Table 1.

**Table 1 The tensile strength of the silicone rubber layer in Example 1**

| Sample number | Tensile strength (MPa) |
|---|---|
| 1 | 9.2 |
| 2 | 9.3 |
| 3 | 9.5 |

The test results show that the silicone rubber layer with the mesh-shaped intermediate layer provided by the present disclosure has excellent mechanical strength. The mesh-shaped intermediate layer is similar to a layer of adjustable reinforcing mesh, which can balance the high strength and flexibility of the entire silicone rubber layer, improve the mechanical properties of the silicone rubber layer, so that the silicone rubber layer has the appropriate fitting performance and good tensile and tearing strength, and is convenient for stitching, and has a close attachment to a wound surface.

### Example 2

This example provides an artificial dermal repair material, and a preparation process thereof is as follows.

At S1, a collagen complex layer was prepared.

Collagen was weighed and dissolved in water to prepare 200 g of a collagen solution with a mass concentration of 0.3%. Then, 0.013 g of chondroitin sulfate was weighed and dissolved in water to prepare a chondroitin sulfate solution with a mass concentration of 0.37 mg/mL. The chondroitin sulfate solution was atomized by high-speed airflow and then added to the collagen solution, and continuously stirred for 2 hours to obtain a collagen-chondroitin sulfate complex. The collagen-chondroitin sulfate complex was centrifuged at 8000 rpm for 15 minutes. The precipitate was collected, and 0.03M acetic acid was added to re-dissolve the precipitate, to make the concentration of collagen 0.9% (w/w), and then stirred for 2.5h to be dispersed uniformly. Then, an acetic acid solution of glutaraldehyde was added to form a collagen complex solution to make the concentration of the collagen 0.7% (w/w), where a mass fraction of the added glutaraldehyde was 0.005% (that is, a mass fraction of the glutaraldehyde accounting for the collagen complex solution), and then stirred for 5h to be uniformly distributed. Then, a PHMB antibacterial agent solution was added to make the concentration of the collagen 0.65% (w/w), where the amount of the added PHMB was 0.0062 g, and then was stirred for 2h to be uniformly distributed, and then was poured into an aluminum alloy mold, leveled, pre-frozen at -60°C for 2h, and then annealed for 5min to 10min, and then frozen at -60°C for half an hour, and then transferred to a freeze dryer for freeze-drying for 24h, and then placed under a high-temperature vacuum condition of 105 °C for high-temperature cross-linking for 24 hours, to obtain the collagen complex layer.

At S2, a silicone rubber layer was prepared.

Components A and B of liquid silicone rubber with normal strength (with tensile strength <2Mpa after film formation) were mixed together in a ratio of 1:1, stirred at 300 rpm for 10 minutes, and then defoamed, and then poured into a polytetrafluoroethylene mold, leveled, and placed in a drying oven and cured at 150°C for 2 hours to obtain a first silicone rubber layer with a thickness of 0.1 mm.

Components A and B of liquid silicone rubber with high strength (with tensile strength >10Mpa after film formation) were mixed together in a ratio of 1:1, stirred at 300 rpm for 10 minutes to form a mixed solution, and then the mixed solution was defoamed. A layer of silicone rubber layer in a parallelogram mesh-shape was uniformly prepared on a surface of the first silicone rubber layer with the mixed solution by using a dispenser, in which a spacing between parallel lines was 1 mm to 10 mm, and then was placed in a drying oven for curing and cured at 150°C for 2 hours, to obtain a second mesh-shaped silicone rubber layer with a thickness of 0.01 mm.

Then, a liquid silicone rubber with normal-strength (with tensile strength <2Mpa after film formation) was leveled on a surface of the second mesh-shaped silicone rubber layer, and then placed in a drying oven, and cured at 150°C for 2 hours, to obtain a third silicone rubber layer with a thickness of 0.1 mm. Finally, a silicone rubber layer with a thickness of 0.21 mm and having a mesh-shaped intermediate layer was formed.

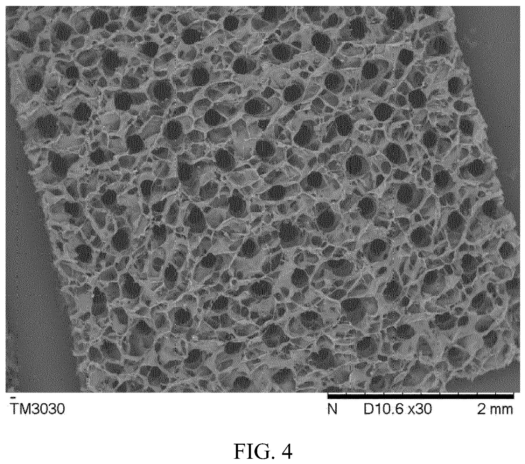

At S3, parameters such as hole spacing, number of rows, and hole size were set on an operation interface of the laser drilling machine. The collagen complex layer obtained in step S1 was placed and fixed in the machine, and the program was run to drill holes. The hole size was 250 µm. The hole spacing was 0.5 mm. The drilled collagen complex layer was characterized, and a SEM image thereof was as shown in FIG. 4.

At S4, a layer of silicone gel was coated on a surface of the silicone rubber layer, and the drilled collagen complex layer was bonded to a surface of the silicone rubber layer to obtain the artificial dermal repair material.

At S5, packaging and sterilizing were performed. The prepared artificial dermal repair material was packaged and sterilized by high-energy electron beam, with a sterilization dose of 15KGy to 20KGy.

### Example 3

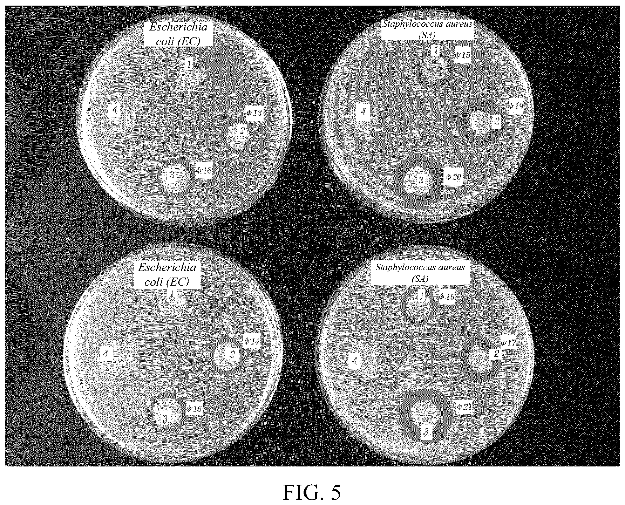

This example provides three types of artificial dermal repair materials, which are the same as that in Example 1. The difference was in that a content of the PHMB was 1.2%, 6%, and 12%, respectively. Bacteriostatic experiments were carried out on the three types of artificial dermal repair materials, and the operation was as follows: the artificial dermal repair materials were cut into discs with a diameter of 12 cm, and sterilized by ultraviolet irradiation for 30 minutes in a super clean bench for standby. A Mueller-Hinton (MH) agar medium was prepared, and then heated to be dissolved completely, and then sterilized at 121°C for 15 minutes, followed by being poured into a dish in a sterile environment when the temperature dropped to 40°C to 50°C, and then cooled and solidified for later use. A sterile cotton swab was used to dip a corrected *Escherichia coli* and *Staphylococcus aureus* bacterial solutions at 0.5 Mcfarland (one Mcfarland = 3^{∗}10⁸ cfu/ml), and then the excess bacterial solution was squeezed off by slightly abutting against a tube wall. The entire surface of the medium was coated by the cotton swab, the plate was rotated by 60 degrees each time, the smearing was carried out three times, and finally, was wiped around a periphery for two circles, to ensure that the bacterial solution was uniformly smeared. The sample was attached on a surface of the plate by sterile forceps, and cannot be taken away after being attached. Four samples were attached to each dish. The plate was turned over and cultured in a 37°C constant temperature incubator for 18 hours to 24 hours, and then taken out for measurement. A diameter of an inhibition zone was measured by a ruler on a back of the plate (measured crosswise, horizontally and vertically, calculating the average value, and making the average value an integer). An edge of the inhibition zone was limited to the fact that no obvious growth of bacteria was visible to the naked eye. The results were as shown in FIG. 5. In the figure, the mark E indicates the *Escherichia coli* bacteriostatic experiment, and the mark S indicates the *Staphylococcus aureus* bacteriostatic experiment. Nos. 1 to 3 correspond to artificial dermal repair materials with a PHMB content of 1.2%, 6%, and 12%, respectively. No. 4 is a blank control group without PHMB. The experimental results show that the artificial dermal repair materials prepared by the present disclosure have bacteriostasis to both *Gram-negative bacteria* (such as *Escherichia coli*) and *Gram-positive bacteria* (such as *Staphylococcus aureus*), and have stronger bacteriostasis to *Gram-positive bacteria.*

### Example 4

This example provides an artificial dermal repair material, and a preparation process thereof is as follows.

At S1, a collagen complex layer was prepared.

Collagen was weighed and dissolved in water to prepare 200 g of a collagen solution with a mass concentration of 0.3%. Then, 0.123 g of chondroitin sulfate was weighed and dissolved in water to prepare a chondroitin sulfate solution with a mass concentration of 3.54 mg/mL. The chondroitin sulfate solution was atomized by high-speed airflow and then added to the collagen solution, and continuously stirred for 2 hours to obtain a collagen-chondroitin sulfate complex. The collagen-chondroitin sulfate complex was centrifuged at 8000 rpm for 15 minutes. The precipitate was collected, and 0.1M acetic acid was added to re-dissolve the precipitate to make the concentration of collagen 0.9% (w/w), and then stirred for 2.5h to be dispersed uniformly. Then, an acetic acid solution of glutaraldehyde was added to form a collagen complex solution to make the concentration of the collagen 0.7% (w/w), where a mass fraction of the added glutaraldehyde was 0.015% (that is, a mass fraction of the glutaraldehyde accounting for the collagen complex solution), and then stirred for 5h to be uniformly distributed. Then, a PHMB antibacterial agent solution was added to make the concentration of the collagen 0.65% (w/w), where the amount of the added PHMB was 0.099 g, and then was stirred for 2h to be uniformly distributed, and then was poured into an aluminum alloy mold, leveled, and pre-frozen at -60°C for 2h, and then annealed for 5min to 10min, and then frozen at - 60°C for half an hour, and then transferred to a freeze dryer for freeze-drying for 24h, and then placed under a high-temperature vacuum condition of 105°C for high-temperature cross-linking for 24 hours, to obtain the collagen complex layer.

At S2, a silicone rubber layer was prepared.

Components A and B of liquid silicone rubber with normal strength (with tensile strength <2Mpa after film formation) were mixed together in a ratio of 1:1, stirred at 300 rpm for 10 minutes, and then defoamed, and then poured into a polytetrafluoroethylene mold, leveled, and placed in a drying oven and cured at 150°C for 2 hours to obtain a first silicone rubber layer with a thickness of 0.15 mm.

Components A and B of liquid silicone rubber with high strength (with tensile strength >10Mpa after film formation) were mixed together in a ratio of 1:1, stirred at 300 rpm for 10 minutes to form a mixed solution, and then the mixed solution was defoamed. A layer of silicone rubber layer in a parallelogram mesh-shape was uniformly prepared on a surface of the first silicone rubber layer with the mixed solution by using a dispenser, in which a spacing between parallel lines was 1 mm to 10 mm, and then was placed in a drying oven for curing and cured at 150°C for 2 hours, to obtain a second mesh-shaped silicone rubber layer with a thickness of 0.05 mm.

Then, a liquid silicone rubber with normal-strength (with tensile strength <2Mpa after film formation) was leveled on a surface of the second mesh-shaped silicone rubber layer, and then placed in a drying oven, and cured at 150°C for 2 hours, to obtain a third silicone rubber layer with a thickness of 0.1 mm. Finally, a silicone rubber layer with a thickness of 0.3 mm and having a mesh-shaped intermediate layer was formed.

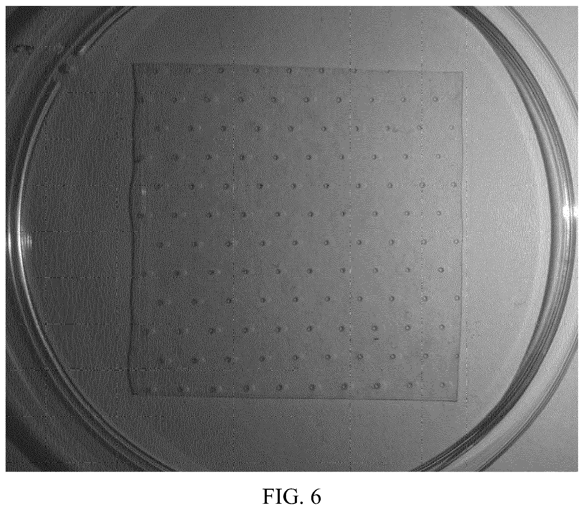

At S3, parameters such as hole spacing, number of rows, and hole size were set on the operation interface of the laser drilling machine. The collagen complex layer obtained in step S1 was placed and fixed in the machine, and the program was run to drill holes. The hole size was 250 µm. The hole spacing was 5 mm. The silicone rubber layer obtained in step S2 was drilled, with a hole size of 500 µm and a hole spacing is 5 mm. The drilled silicone rubber layer was as shown in FIG. 6.

At S4, a layer of silicone gel was coated on a surface of the silicone rubber layer, and the drilled collagen complex layer was bonded to a surface of the drilled silicone rubber layer to obtain the artificial dermal repair material.

At S5, packaging and sterilizing were performed. The prepared artificial dermal repair material was packaged and sterilized by high-energy electron beam, with a sterilization dose of 15KGy to 20KGy.

## Claims

1. An artificial dermal repair material, comprising a silicone rubber layer and a collagen complex layer bonded to each other, the collagen complex layer being prepared by raw materials comprising collagen, glycosaminoglycan, and an antibacterial agent by a cross-linking reaction,
wherein the silicone rubber layer comprises a first silicone rubber layer, a second mesh-shaped silicone rubber intermediate layer, and a third silicone rubber layer which are provided in sequence, a tensile strength of the second mesh-shaped silicone rubber intermediate layer is greater than a tensile strength of the first silicone rubber layer and a tensile strength of the third silicone rubber layer.

2. The artificial dermal repair material according to claim 1, wherein the tensile strength of the first silicone rubber layer and/or the third silicone rubber layer is less than 2 MPa, the tensile strength of the second mesh-shaped silicone rubber intermediate layer is greater than 10 MPa.

3. The artificial dermal repair material according to claim 1, wherein the collagen complex layer is of a gradient porous structure.

4. The artificial dermal repair material according to any one of claims 1 to 3, wherein the raw materials comprise 73% to 97% of the collagen, 2% to 15% of the glycosaminoglycan, and 1% to 12% of the antibacterial agent.

5. The artificial dermal repair material according to claim 4, wherein the raw materials comprise 78% to 93% of the collagen, 3% to 10% of the glycosaminoglycan, and 4% to 12% of the antibacterial agent.

6. The artificial dermal repair material according to any one of claims 1 to 3 and claim 5, wherein at least one of the silicone rubber layer and the collagen complex layer is provided with a plurality of holes.

7. A method of preparing an artificial dermal repair material according to any one of claims 1 to 6, comprising:
preparing a collagen complex layer, comprising:
dissolving glycosaminoglycan in a solvent to obtain a glycosaminoglycan solution, and dissolving collagen in a solvent to obtain a collagen solution;
mixing the glycosaminoglycan solution and the collagen solution, performing centrifugation and collecting a precipitate, and redissolving the precipitate by adding acetic acid to obtain a collagen-glycosaminoglycan complex solution; and
adding a cross-linking agent and an antibacterial agent into the collagen-glycosaminoglycan complex solution, performing freeze-drying, and then further performing a cross-linking reaction under a vacuum condition to obtain the collagen complex layer;
preparing a silicone rubber layer,
bonding the collagen complex layer and the silicone rubber layer, and
optionally preparing a hole on at least one of the collagen complex layer and the silicone rubber layer;
wherein the preparing the silicone rubber layer comprises:
mixing and curing materials of a first silicone rubber to form a first silicone rubber layer;
mixing materials of a second silicone rubber to form a mixed solution, preparing a mesh-shaped layer on the first silicone rubber layer with the mixed solution by using a dispenser, and performing curing to form a second mesh-shaped silicone rubber intermediate layer; and
leveling materials of a third silicone rubber on a surface of the second mesh-shaped silicone rubber intermediate layer, and performing curing to form a third silicone rubber layer;
wherein a tensile strength of the materials of the second silicone rubber after film formation is greater than a tensile strength of the materials of the first silicone rubber and a tensile strength of the materials of the third silicone rubber after film formation.

8. The method of preparing the artificial dermal repair material according to claim 7, wherein the performing freeze-drying comprises: performing freezing under a low temperature environment, annealing for 5min to 10min, and then freezing under a low temperature environment, and then freeze-drying in a freeze dryer.

9. The method of preparing the artificial dermal repair material according to claim 7, wherein the cross-linking agent is one selected from the group consisting of glutaraldehyde, genipin, and carbodiimide.

## Patentansprüche

1. Künstliches Hautreparaturmaterial, das eine Silikongummischicht und eine Kollagenkomplexschicht, die aneinander gebunden sind, umfasst, wobei die Kollagenkomplexschicht durch Rohmaterialien, die Kollagen, Glykosaminoglykan und ein antibakterielles Mittel umfassen, durch eine Vernetzungsreaktion hergestellt wird,
wobei die Silikongummischicht eine erste Silikongummischicht, eine zweite maschenförmige Silikongummi-Zwischenschicht und eine dritte Silikongummischicht umfasst, die nacheinander bereitgestellt werden, wobei eine Zugfestigkeit der zweiten maschenförmigen Silikongummi-Zwischenschicht größer ist als eine Zugfestigkeit der ersten Silikongummischicht und eine Zugfestigkeit der dritten Silikongummischicht.

2. Künstliches Hautreparaturmaterial nach Anspruch 1, wobei die Zugfestigkeit der ersten Silikongummischicht und/oder der dritten Silikongummischicht weniger als 2 MPa beträgt, die Zugfestigkeit der zweiten maschenförmigen Silikongummi-Zwischenschicht größer als 10 MPa ist.

3. Künstliches Hautreparaturmaterial nach Anspruch 1, wobei die Kollagenkomplexschicht eine gradientporöse Struktur aufweist.

4. Künstliches Hautreparaturmaterial nach einem der Ansprüche 1 bis 3, wobei die Rohmaterialien 73 % bis 97 % des Kollagens, 2 % bis 15 % des Glykosaminoglykans und 1 % bis 12 % des antibakteriellen Mittels umfassen.

5. Künstliches Hautreparaturmaterial nach Anspruch 4, wobei die Rohmaterialien 78% bis 93% des Kollagens, 3% bis 10% des Glykosaminoglykans und 4% bis 12 % des antibakteriellen Mittels umfassen.

6. Künstliches Hautreparaturmaterial nach einem der Ansprüche 1 bis 3 und 5, wobei mindestens eine der Silikonkautschukschicht und der Kollagenkomplexschicht mit einer Vielzahl von Löchern versehen ist.

7. Verfahren zur Herstellung eines künstlichen Hautreparaturmaterials nach einem der Ansprüche 1 bis 6, das Folgendes umfasst:
Herstellen einer Kollagenkomplexschicht, die Folgendes umfasst:
Auflösen von Glykosaminoglykan in einem Lösungsmittel, um eine Glykosaminoglykanlösung zu erhalten, und Auflösen von Kollagen in einem Lösungsmittel, um eine Kollagenlösung zu erhalten;
Mischen der Glykosaminoglykanlösung und der Kollagenlösung, Durchführen einer Zentrifugation und Sammeln eines Präzipitats und Wiederauflösen des Präzipitats durch Zugabe von Essigsäure, um eine Kollagen-Glykosaminoglykan-Komplexlösung zu erhalten; und
Zugeben eines Vernetzungsmittels und eines antibakteriellen Mittels in die Kollagen-Glykosaminoglykan-Komplexlösung, Durchführen einer Gefriertrocknung und dann weiteres Durchführen einer Vernetzungsreaktion unter einer Vakuumbedingung, um die Kollagen-Komplexschicht zu erhalten;
Herstellen einer Silikonkautschukschicht,
Verbinden der Kollagenkomplexschicht und der Silikongummischicht, und
optional Herstellen eines Lochs auf mindestens einer der Kollagenkomplexschicht und der Silikongummischicht;
wobei die Herstellung der Silikongummischicht Folgendes umfasst:
Mischen und Aushärten von Materialien eines ersten Silikongummis, um eine erste Silikongummischicht zu bilden;
Mischen von Materialien eines zweiten Silikongummis zur Bildung einer gemischten Lösung, Herstellen einer netzförmigen Schicht auf der ersten Silikongummischicht mit der gemischten Lösung unter Verwendung eines Dispensers, und Durchführen des Aushärtens zur Bildung einer zweiten maschenförmigen Silikongummi-Zwischenschicht; und
Ausgleichen von Materialien eines dritten Silikongummis auf einer Oberfläche der zweiten maschenförmigen Silikongummi-Zwischenschicht und Durchführen des Aushärtens zur Bildung einer dritten Silikongummischicht;
wobei eine Zugfestigkeit der Materialien des zweiten Silikongummis nach der Filmbildung größer ist als eine Zugfestigkeit der Materialien des ersten Silikongummis und eine Zugfestigkeit der Materialien des dritten Silikongummis nach der Filmbildung.

8. Verfahren zur Herstellung des künstlichen Hautreparaturmaterials nach Anspruch 7, wobei das Durchführen der Gefriertrocknung Folgendes umfasst: Durchführen des Einfrierens in einer Umgebung mit niedriger Temperatur, Ausglühen für 5 Minuten bis 10 Minuten, und dann Einfrieren in einer Umgebung mit niedriger Temperatur, und dann Gefriertrocknen in einem Gefriertrockner.

9. Verfahren zur Herstellung des künstlichen Hautreparaturmaterials nach Anspruch 7, wobei das Vernetzungsmittel eines ist, das ausgewählt ist aus der Gruppe bestehend aus Glutaraldehyd, Genipin und Carbodiimid.

## Revendications

1. Matériau artificiel de réparation dermique, comprenant une couche de caoutchouc de silicone et une couche de complexe de collagène liées l'une à l'autre, la couche de complexe de collagène étant préparée par des matières premières comprenant du collagène, du glycosaminoglycane et un agent antibactérien par une réaction de réticulation,
dans lequel le couche de caoutchouc de silicone comprend une première couche de caoutchouc de silicone, une deuxième couche intermédiaire de caoutchouc de silicone en forme de maille et une troisième couche de caoutchouc de silicone qui sont fournies en séquence, une résistance à la traction de la deuxième couche intermédiaire de caoutchouc de silicone en forme de maille est supérieure à une résistance à la traction de la première couche de caoutchouc de silicone et à une résistance à la traction de la troisième couche de caoutchouc de silicone.

2. Matériau artificiel de réparation dermique selon la revendication 1, dans lequel la résistance à la traction de la première couche de caoutchouc de silicone et/ou de la troisième couche de caoutchouc de silicone est inférieure à 2 MPa, la résistance à la traction de la deuxième couche intermédiaire de caoutchouc de silicone en forme de maille est supérieure à 10 MPa.

3. Matériau artificiel de réparation dermique selon la revendication 1, dans lequel la couche de complexe de collagène est d'une structure poreuse à gradient.

4. Matériau artificiel de réparation dermique selon l'une quelconque des revendications 1 à 3, dans lequel les matières premières comprennent 73 % à 97 % du collagène, 2 % à 15 % du glycosaminoglycane et 1 % à 12 % de l'agent antibactérien.

5. Matériau artificiel de réparation dermique selon la revendication 4, dans lequel les matières premières comprennent 78 % à 93 % du collagène, 3 % à 10 % du glycosaminoglycane et 4 % à 12 % de l'agent antibactérien.

6. Matériau artificiel de réparation dermique selon l'une quelconque des revendications 1 à 3 et la revendication 5, dans lequel au moins l'une parmi la couche de caoutchouc de silicone et la couche de complexe de collagène est pourvue d'une pluralité de trous.

7. Procédé de préparation d'un matériau artificiel de réparation dermique selon l'une quelconque des revendications 1 à 6, comprenant :
la préparation d'une couche de complexe de collagène, comprenant :
la dissolution du glycosaminoglycane dans un solvant pour obtenir une solution de glycosaminoglycane, et la dissolution du collagène dans un solvant pour obtenir une solution de collagène ;
le mélange de la solution de glycosaminoglycane et de la solution de collagène, la réalisation d'une centrifugation et le recueil d'un précipité, et la redissolution du précipité en ajoutant de l'acide acétique pour obtenir une solution de complexe collagène-glycosaminoglycane ; et
l'ajout d'un agent de réticulation et d'un agent antibactérien dans la solution de complexe collagène-glycosaminoglycane, la réalisation d'une lyophilisation, puis la réalisation en outre d'une réaction de réticulation sous vide pour obtenir la couche de complexe de collagène ;
la préparation d'une couche de caoutchouc de silicone,
la liaison de la couche de complexe de collagène et de la couche de caoutchouc de silicone, et
éventuellement la préparation d'un trou sur au moins l'une parmi la couche de complexe de collagène et la couche de caoutchouc de silicone ;
dans lequel la préparation de la couche de caoutchouc de silicone comprend :
le mélange et le durcissement des matériaux d'un premier caoutchouc de silicone pour former une première couche de caoutchouc de silicone ;
le mélange des matériaux d'un deuxième caoutchouc de silicone pour former une solution mixte, la préparation d'une couche en forme de maille sur la première couche de caoutchouc de silicone avec la solution mixte en utilisant un distributeur, et la réalisation d'un durcissement pour former une deuxième couche intermédiaire de caoutchouc de silicone en forme de maille ; et
le nivellement des matériaux d'un troisième caoutchouc de silicone sur une surface de la deuxième couche intermédiaire de caoutchouc de silicone en forme de maille, et la réalisation d'un durcissement pour former une troisième couche de caoutchouc de silicone ;
dans lequel une résistance à la traction des matériaux du deuxième caoutchouc de silicone après la formation de film est supérieure à une résistance à la traction des matériaux du premier caoutchouc de silicone et à une résistance à la traction des matériaux du troisième caoutchouc de silicone après formation de film.

8. Procédé de préparation du matériau artificiel de réparation dermique selon la revendication 7, dans lequel la réalisation de la lyophilisation comprend : la réalisation de la congélation dans un environnement à basse température, un recuit pendant 5 min à 10 min, puis la congélation dans un environnement à basse température, et puis la lyophilisation dans un lyophilisateur.

9. Procédé de préparation du matériau artificiel de réparation dermique selon la revendication 7, dans lequel l'agent de réticulation est un agent choisi dans le groupe constitué du glutaraldéhyde, de la génipine et du carbodiimide.
